(19) **Europäisches Patentamt / European Patent Office / Office européen des brevets**

(11) **EP 1 704 825 B1**

(12) **EUROPÄISCHE PATENTSCHRIFT**

(45) Veröffentlichungstag und Bekanntmachung des Hinweises auf die Patenterteilung:
**08.02.2012 Patentblatt 2012/06**

(51) Int Cl.:
***A61B 19/00*** *(2006.01)*

(21) Anmeldenummer: **05006219.9**

(22) Anmeldetag: **22.03.2005**

(54) **Führungsdrahtnavigation**

Guide wire navigation

Navigation de fil de guidage

(84) Benannte Vertragsstaaten:
**DE FR GB IT**

(43) Veröffentlichungstag der Anmeldung:
**27.09.2006 Patentblatt 2006/39**

(73) Patentinhaber: **BrainLAB AG**
**85622 Feldkirchen (DE)**

(72) Erfinder: **Maier, Christian**
**81827 München (DE)**

(74) Vertreter: **Schwabe - Sandmair - Marx**
**Patentanwälte**
**Stuntzstraße 16**
**81677 München (DE)**

(56) Entgegenhaltungen:
**EP-A- 1 413 257         WO-A-03/043485**
**DE-A1- 10 118 570      US-A- 5 274 551**
**US-A1- 2003 229 279**

**Beschreibung**

Die vorliegende Erfindung betrifft das technische Gebiet der Navigation eines Führungsdrahtes. Führungsdrähte werden verwendet, um nach einem Knochenbruch Knochenfragmente "aufzufädeln", bevor beispielsweise ein Marknagel eingebracht wird. Der Führungsdraht ist ein langer, flexibler und relativ dünner Stab mit einem proximalen Ende, an dem der Führungsdraht gegriffen werden kann, und mit einem distalen Ende, das durch den Knochenkanal bzw. die Kanäle der Knochenfragmente hindurch nach vorne gebracht wird, um die Fragmente aufzufädeln.

Es wäre bei dieser Tätigkeit von großem Vorteil, wenn man jederzeit wüsste, wo das distale Ende des Führungsdrahtes sich befindet. Man könnte dann immer feststellen, ob dieses distale Ende schon weit genug vorgebracht wurde, um ein ausreichendes Auffädeln zu gewährleisten.

Derzeit wird eine Verfolgung des proximalen Endes bzw. des gesamten Führungsdrahtes in einem Röhrenknochen mit Hilfe fluoroskopischer Aufnahmen (Röntgenaufnahmen) durchgeführt. Dabei wird die Lage des Führungsdrahtes unter Verwendung eines C-Bogen-Röntgengerätes visualisiert und dabei natürlich auch die Lage des distalen Endes gegenüber dem Knochen bzw. den Knochenfragmenten. Die hierbei notwendigen relativ zahlreichen Einzel-Röntgenaufnahmen oder gar Dauer-Röntgenaufnahmen bringen eine starke Strahlenbelastung sowohl für den Patienten als auch für den Behandelnden mit sich.

Beim gezielten Einsetzen von Kanülen oder Kathetern ist in der Medizintechnik vorgeschlagen worden, eine Magnetfeldnavigation zu verwenden. Beispiele für Magnetfeldnavigationen sind in der US 6,104,944 und der US 6,783,536 B2 aufgezeigt. Eine Magnetfeldnavigation oder ähnlich wirkende Navigationsarten, die innerhalb eines Körpers verdeckte Instrumente navigieren oder positionell erfassen, sind aber technisch aufwändig und störanfällig.

Optische Navigationssysteme sind bekannt, beispielsweise aus der DE 196 39 615 A1, auf deren Offenbarung hier ergänzend Bezug genommen wird. Es ist aber bisher nicht für möglich gehalten worden, einen Instrumentenabschnitt eines flexiblen Instruments, der innerhalb eines Patientenkörpers liegt, mittels eines optischen Navigations- und Trackingsystems positionell zu bestimmen. Dies liegt einfach daran, dass man den Verlauf des flexiblen Instruments nicht für erfassbar hielt und eine optische Referenzanordnung an dem flexiblen Teil bzw. an der Spitze des flexiblen Teils nicht sinnvoll angeordnet werden konnte, da dieser Teil sich innerhalb der Körperstrukturen befindet.

Aus der DE 101 18 570 A1 ist eine medizintechnische Vorrichtung mit einem flexiblen Knochenführungsdraht bekannt, dessen Position, Ausrichtung und Form mittels eines Navigationssystems und eines faseroptischen Positionsgebers bestimmbar ist. Am proximalen Ende des Knochenführungsdrahtes ist eine Referenzvorrichtung angeordnet, deren Position und Ausrichtung und somit auch die Position und Ausrichtung des proximalen Endes des Knochenführungsdrahtes mittels des Navigationssystems erfasst wird. Um die Form des Knochenführungsdrahtes und letztlich auch die Position und Ausrichtung der näher am distalen Ende gelegenen Teile des Knochenführungsdrahtes zu bestimmen, ist in den Knochenführungsdraht ein faseroptischer Positionsgeber mit optischen Fasern eingearbeitet, aus denen je nach Stärke der Biegung der Fasern Licht austreten kann. Das austretende Licht wird erfasst und zusammen mit den Daten über die Position und Ausrichtung des proximalen Endes des Knochenführungsdrahtes einer Datenverarbeitungsanlage zur Berechnung der Position, Ausrichtung und Form des Knochenführungsdrahtes zugeführt. Der faseroptische Positionsgeber ist allerdings anfällig gegenüber mechanischen Schäden. Wird die reflektierende Oberfläche einzelner Fasern beschädigt, hat dies Auswirkungen auf das von den Fasern emittierte Licht, was wiederum zu fehlerhaften Messungen und zu einer falschen Berechnung der Form des Knochenführungsdrahtes führen kann.

Es ist die Aufgabe der vorliegenden Erfindung, das Ermitteln des distalen Endes eines Knochenführungsdrahtes zu ermöglichen, ohne dass der Patient und der Behandelnde starken Strahlungsdosen ausgesetzt werden und ohne einen großen apparativen Aufwand, z.B. mit magnetischen Navigations- und Trackingsystemen, treiben zu müssen. Ferner ist es insbesondere eine Aufgabe der vorliegenden Erfindung, die Positionsermittlung einfach und mit bereitstehenden Mitteln zu ermöglichen sowie möglichst störungsunanfällig.

Diese Aufgabe wird durch ein Verfahren gemäß dem Patentanspruch 1, durch ein Bildunterstützungsverfahren gemäß dem Patentanspruch 3, durch eine Vorrichtung gemäß dem Patentanspruch 4, durch ein Programm gemäß dem Patentanspruch 7 und durch ein Computerprogramm-Speichermedium gemäß dem Patentanspruch 8 gelöst. Die Unteransprüche definieren bevorzugte Ausführungsformen der vorliegenden Erfindung.

Die Erfindung stellt somit ein Verfahren zum Ermitteln der Position des distalen Endes eines Knochenführungsdrahtes zur Verfügung, bei dem

- mit Hilfe eines medizintechnischen, optischen Tracking- und Navigationssystems und einer Referenzvorrichtung am proximalen Ende des Führungsdrahtes die Position und Ausrichtung des proximalen Endes des Führungsdrahtes ermittelt wird;
- mit Hilfe des medizintechnischen, optischen Tracking- und Navigationssystems und einer Referenzvorrichtung an einem Knochen, in dem sich das distale Ende des Führungsdrahtes befindet, die Ausrichtung des Knochens ermittelt wird; und bei dem

(a) mittels der Randbedingungen für den Führungsdraht-Verlauf, die durch die Position und Ausrichtung des proximalen Endes und die Ausrichtung des Knochens gegeben werden, und

(b) unter Berücksichtigung der körperlichen Eigenschaften des Führungsdrahtes computergestützt die Position des distalen Endes des Knochenführungsdrahtes ermittelt wird.

Bei dem Verfahren kann die computerunterstützte Positionsermittlung durch den Computer des Tracking- und Navigationssystems durchgeführt werden.

Das erfindungsgemäße Bildunterstützungsverfahren für die Verwendung eines Knochenführungsdrahtes ermittelt die Position des distalen Endes des Knochenführungsdrahtes gemäß dem oben beschriebenen Verfahren. Außerdem wird diese Position auf einer Bildausgabe des Tracking- und Navigationssystems in Relation zur Knochenanordnung ausgegeben.

Die Erfindung betrifft im Einzelnen ferner eine Vorrichtung zum Ermitteln der Position des distalen Endes eines Knochenführungsdrahtes, mit einem medizintechnischen, optischen Tracking- und Navigationssystems, mit einer Referenzvorrichtung am proximalen Ende des Führungsdrahtes und mit einer Referenzvorrichtung an einem Knochen, in dem sich das distale Ende des Führungsdrahtes befindet, und mit einem Computer, der

(a) mittels der Randbedingungen für den Führungsdraht-Verlauf, die durch die Position und Ausrichtung des proximalen Endes und die Ausrichtung des Knochens gegeben werden, und

(b) unter Berücksichtigung der körperlichen Eigenschaften des Führungsdrahtes die Position des distalen Endes des Knochenführungsdrahtes ermittelt.

Der Computer kann der Computer des Tracking- und Navigationssystems sein. Das Tracking- und Navigationssystem kann eine Bildausgabe aufweisen, welche die ermittelte Position des distalen Endes in Relation zur Knochenanordnung ausgibt.

Die Erfindung wird im Weiteren mit Hilfe der beiliegenden Zeichnungen näher erläutert. Es zeigen:

Figur 1    eine Abbildung des Umfeldes eines erfindungsgemäß navigierten Führungsdrahtes; und
Figur 2    eine schematische Darstellung des Führungsdrahtes zum Zwecke der theoretischen Erörterung.

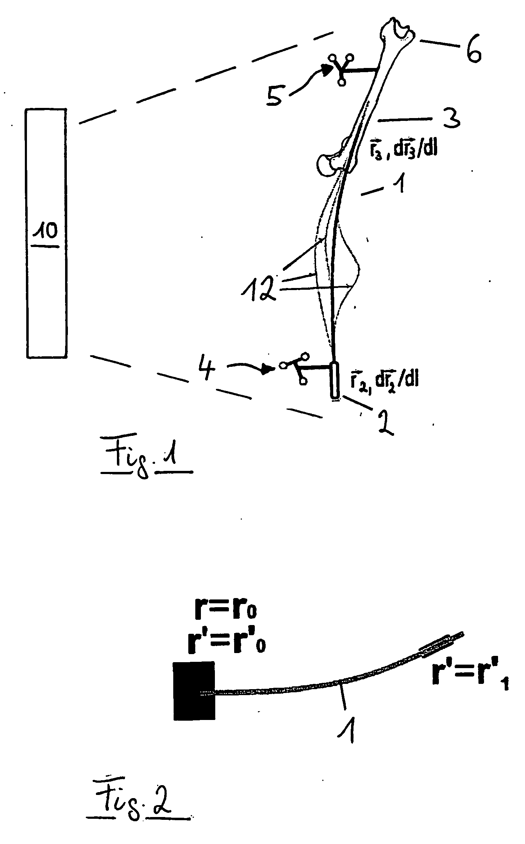

Der in Figur 1 dargestellte Führungsdraht 1 weist an seinem proximalen Ende 2 einen Handgriff auf, an dem eine Navigations-Referenzvorrichtung 4 befestigt ist. Die Navigations-Referenzvorrichtung 4 sowie die Referenzvorrichtung 5, die an dem dargestellten Knochen 6 fest angeordnet ist, sind von dem nur schematisch dargestellten Navigations- und Trackingsystem 10 positionell erfassbar. Zu dem System 10 können beispielsweise Trackingkameras, eine Datenverarbeitungsanlage sowie eine Bildausgabe und Dateneingabemittel gehören. Das System 10 kann ein übliches optisches Navigations- und Trackingsystem sein; die Referenzvorrichtungen 4 und 5 sind als Reflektorensterne ausgebildet, die unsichtbares (infrarotes) Licht reflektieren. Insbesondere kann das Navigationssystem ein solches sein, wie es beispielsweise als "Neuronavigationssystem" in der DE 196 39 615 A1 beschrieben ist.

Das Navigations- und Trackingsystem kann die Position und die Ausrichtung des proximalen Endes 2 ermitteln ($r_2$, $dr_2/dl$). Auch kann die Position und Ausrichtung des Knochens 6 ermittelt werden, und insbesondere die Ausrichtung des inneren Röhrenkanals im Knochen 6. Gesucht wird der Vektor $r_3$, das heißt die Position des distalen Endes 3 des Führungsdrahtes 1.

Die Erfindung ist mit den vorher schon beschriebenen Schritten dazu in der Lage, die Einsetztiefe des distalen Endes 3 unter bestimmten Bedingungen zu ermitteln. Hierzu wird entgegen allen bisherigen Ansätzen im Stand der Technik (Magnetnavigation) eine optische Navigation verwendet. Der Handgriff des Führungsdrahtes (distales Ende 2) kann optisch ohne weiteres getrackt werden, und wegen der bekannten Knochenausrichtung (Referenzvorrichtung 5) ist die Ausrichtung der Achse des Röhrenknochens bekannt, in welche der Führungsdraht eingesetzt wird. Dies gestattet es, die Einsetztiefe des Führungsdrahtes auf einem Computermonitor virtuell darzustellen.

Die Erfindung berücksichtigt die starke Verbiegung des flexiblen Führungsdrahtes. Dies ist möglich, weil die Ausrichtung des Führungsdrahtes im Bereich der Spitzenposition bekannt ist. Dieses Wissen kann verwendet werden, um die Biegung zu berücksichtigen, die ansonsten nicht erfasst werden könnte. Es sind theoretisch sehr viele Konturen möglich, und in Figur 1 sind einige davon mit dem Bezugszeichen 12 aufgezeigt. Bisher wurden optische Navigationssysteme nur dazu verwendet, starre Körper zu verfolgen und darzustellen, bei welchen die Position und Ausrichtung an einem einzigen Punkt die Position und Ausrichtung der gesamten Struktur bestimmt. Die hier beschriebene Erfindung gestattet es, die Spitze bzw. das distale Ende eines flexiblen Führungsdrahtes zu tracken bzw. zu verfolgen, ohne dass dafür eine invasive Bilderfassungsmethode verwendet werden muss (Rönten-C-Bogen).

Es soll hier ausdrücklich darauf hingewiesen werden, dass die vorliegende Erfindung und die vorliegende Offenbarung grundsätzlich das Ermitteln der Position des distalen Ende jedwedes flexiblen länglichen Instrumentes ermöglichen, wenn die hierin beschriebenen Bedingungen eingehalten werden. Insbesondere ist lediglich erforderlich, dass die Ausrichtung im Bereich des distalen Endes bekannt ist (also hier z.B. jedweder Kanal vorhanden ist, dessen Ausrichtung man kennt), da das andere, proximale Ende optisch trackbar ist (in Position und Ausrichtung) und da das flexible Instrument bestimmte körperliche Eigenschaften aufweist, die im Einzelnen noch unten erläutert werden.

Das Navigationssystem verfolgt und zeigt also die Einsetztiefe für einen flexiblen medizinischen bzw. chirurgischen Draht in einem Röhrenknochen. Das Navigationssystem besteht, obwohl in Figur 1 nicht separat dargestellt, aus einem Kamerasystem, das die Position der Referenzmarkeranordnung 4, die am Handgriff des Führungsdrahtes 1 befestigt ist, und einer Referenzmarkeranordnung 5, die an dem Röhrenknochen angeordnet ist, verfolgt, bzw. trackt. Die Navigationssoftware ist dazu in der Lage, die dreidimensionale Position und Orientierung einer Achse der dreidimensionalen Position und Orientierung der Referenzmarkeranordnungen 4, 5 zuzuordnen. Mit dieser Information ist es möglich, Grenz- bzw. Randbedingungen für die Form des Führungsdrahtes zu definieren, so dass festgestellt werden kann, wie weit der Führungsdraht in den Knochen eingedrungen ist. Gleiches gilt natürlich für größere Knochenfragmente, die nach einem Bruch auftreten können, wenn die Fragmente (oder eines der Fragmente) mit Referenzmarkeranordnungen versehen sind.

Um die Eindringtiefe eines flexiblen Knochenführungsdrahtes zu tracken und darzustellen müssen die folgenden Bedingungen erfüllt sein:

a) mit den Begriffen der Elastizitätstheorie ausgedrückt kann der Knochenführungsdraht als steifer bzw. starrer Stab angesehen werden. "Steif" bedeutet, dass die Persistenzlänge des Drahtes (Lp) sehr viel größer ist als die Länge des Drahtes (L). Die Persistenzlänge ist definiert als der Abstand zweier Punkte auf der Kontur des Drahtes, für welche die Auto-Korrelationsfunktion des Tangentenwinkels der Kontur auf den 1/e-ten Teil abgefallen ist (e = Eulerzahl). Aus der Polymertheorie ist es bekannt, dass die Persistenzlänge geschrieben werden kann als: $L_p = YI/k_B,T$ ($Y$ = Young-Modul, $I$ = Flächenträgheitsmoment des Drahtquerschnitts, $k_B$ = Bolzmannkonstante; $T$ = Temperatur). Mit typischen Werten für $Y$, $I$ und $T$ zeigt eine einfache Rechnung, dass $L_p \gg L$ ist.

b) Die Länge des Drahtes ist konstant. Da der Young-Modul für das Material des Führungsdrahtes bei ungefähr 100 GPa liegt, kann leicht gezeigt werden, dass die auftretenden Kräfte nur zu relativen Längenänderungen von 0,001 und weniger führen.

Bei einer Berechnungsart kann die Kontur des Drahtes als vektorielle Differentialgleichung der dritten Ordnung beschrieben werden. Um diese Gleichung zu lösen, müssen drei vektorielle Randbedingungen bekannt sein, und diese sind:

1) Position des proximalen Drahtendes 2 (am Handgriff)
2) Ausrichtung des proximalen Drahtendes;
3) Ausrichtung eines Punktes auf dem Draht nahe dem distalen Drahtende.

Aus 1) bis 3) können die Randbedingungen definiert und die Differentialgleichung gelöst werden und zwar durch numerische Verfahren. Zusammen mit den Bedingungen a) und b) können relevante Informationen über die Eindringtiefe des Drahtes extrahiert und auf der Bilddarstellung des Navigationssystems angezeigt werden. Bildlich gesprochen gestattet es das Wissen um die Ausrichtung des Führungsdrahtes an einer Position in der Nähe seines eingesetzten Endes (distalen Endes) die korrekte Kontur aus der Vielzahl der möglichen Konturen herauszufinden (die möglichen Konturen sind die in Figur 1 mit 12 gezeigten möglichen Konturen, deren distaler Punkt zwar im Knochen liegt, die jedoch insgesamt anders verlaufen).

Eine etwas detailliertere Beschreibung der elastizitätstheoretischen Grundlagen ist im Folgenden gegeben, wobei die Gleichungen (1.9) und (1.10) auf die Figur 2 Bezug nehmen.

Das vollständige System der Gleichgewichtsbedingungen eines beliebig gebogenen Stabes lautet:

$$\frac{d\boldsymbol{F}}{dl} = -\boldsymbol{K} \qquad\qquad (1.1)$$

$$\frac{d\boldsymbol{M}}{dl} = \boldsymbol{F} \times \boldsymbol{t} \qquad\qquad (1.2)$$

wobei $F$ die inneren Spannungskräfte bezeichnet, $K$ sei die auf den Stab wirkende äußere Kraft,

$M$ ist das Drehmoment der inneren Spannungen, die auf die Querschnittsfläche wirken, $t$ ist der Einheitsvektor der Tangente an den Stab, 1 ist die Bogenlänge auf dem Stab, x bezeichnet das Vektorprodukt.

Die x-Komponente von Gleichung (1.2) ist

$$\frac{dM_x}{dl} = F_y t_z - F_z t_y \qquad (1.3)$$

Leitet man diese Gleichung zweimal nach der Variablen 1 ab, so erhält man zwei weitere Gleichungen, mit deren Hilfe man $F_y$ und $F_z$ eliminieren kann. Außerdem lassen sich die Ableitungen $dF_y/dl$ und $dF_z/dl$ mittels (1.1) durch die Komponenten der äußeren Kraft ausdrücken. Man erhält somit für $M_x$:

$$M_x''' = \left( M_x'' - \frac{t_z'}{t_z} M_x' + K_y t_z - K_z t_y \right) \left( \frac{t_y t_z'' - t_y'' t_z}{t_y t_z' - t_y' t_z} \right) + \frac{t_z''}{t_z} M_x' - K_y' t_x - 2K_y t_x' + K_z' t_y + 2K_z t_y' \qquad (1.4)$$

Analoge Gleichungen folgen für $M_y$ und $M_z$, wenn man die Vertauschung x→y, y→z, z→x einmal bzw. zweimal vornimmt.

Wirken die äußeren Kräfte nur auf einzelne isolierte Punkte, so gilt in den Stababschnitten zwischen den Angriffspunkten der äußeren Kräfte $K = 0$, und damit vereinfacht sich Gleichung (1.4) zu:

$$M_x''' = \left( M_x'' - \frac{t_z'}{t_z} M_x' \right) \left( \frac{t_y t_z'' - t_y'' t_z}{t_y t_z' - t_y' t_z} \right) + \frac{t_z''}{t_z} M_x' \qquad (1.5)$$

Geht man von der Voraussetzung aus, daß der Querschnitt des Stabes kreisförmig ist, so läßt sich das Drehmoment schreiben als:

$$M = EI\, t \times \frac{dt}{dl} \qquad (1.6)$$

mit E-Elastizitätsmodul des Stabmaterials, 1-Flächenträgheitsmoment des Stabquerschnitts ($I = \pi R^4$ für einen kreisrunden Querschnitt mit Radius R).

Einsetzen der x-Komponente von (1.6) in (1.5) ergibt eine DGL der Form:

$$f_1 \left( \frac{d^4 t_y}{dl^4}, \frac{d^4 t_z}{dl^4}, \frac{d^3 t_y}{dl^3}, \frac{d^3 t_z}{dl^3}, \frac{d^2 t_y}{dl^2}, \frac{d^2 t_z}{dl^2}, \frac{dt_y}{dl}, \frac{dt_z}{dl}, t_y, t_z \right) = 0 \qquad (1.7)$$

Mit $t = \frac{dr}{dl}$ ($r$ = Radiusvektor vom Koordinatenursprung zu einem beliebigen Punkt auf dem Stab) ergibt sich aus (1.7) eine DGL der Form:

$$g_1 \left( \frac{d^5 y}{dl^5}, \frac{d^5 z}{dl^5}, \frac{d^4 y}{dl^4}, \frac{d^4 z}{dl^4}, \frac{d^3 y}{dl^3}, \frac{d^3 z}{dl^3}, \frac{d^2 y}{dl^2}, \frac{d^2 z}{dl^2}, \frac{dy}{dl}, \frac{dz}{dl} \right) = 0 \qquad (1.8)$$

Neben $g_1$ gibt es noch zwei weitere DGLn ($g_2$ und $g_3$), die durch die o.g. Indexvertauschung entstehen. Man hat zusammengefaßt ein gekoppeltes DGL-System, in dem die Variable 1 sowie die Funktionen x(1), y(1) und z(1) nicht explizit vorkommen. Demzufolge läßt sich die Ordnung der DGLn um zwei reduzieren auf ein gekoppeltes DGL-System 3. Ordnung bzw. eine Vektordifferentialgleichung 3. Ordnung. Zur Lösung benötigt man drei vektorielle Randbedingungen.

Randbedingungen:

Der Stab ist einseitig fest eingespannt, d.h. es gilt:

$$\boldsymbol{r}(\mathrm{l}=0) = \boldsymbol{r}_0$$
$$\left.\frac{\mathrm{d}\boldsymbol{r}}{\mathrm{d}\mathrm{l}}\right|_{(\mathrm{l}=0)} = \boldsymbol{r}_0' \qquad\qquad (1.9)$$

Der Stab bewegt sich an einer Stelle im Raum $(r=r_1)$ parallel zu einer "Röhre", d.h. an diesem Punkt ist seine Richtung vorgegeben. Der Punkt auf dem Stab, für den diese Bedingung gilt, ist nicht bekannt, jedoch läßt sich diese Randbedingung

$$\left.\frac{\mathrm{d}\boldsymbol{r}}{\mathrm{d}\mathrm{l}}\right|_{(r=r_1)} = \boldsymbol{r}_1' \qquad\qquad (1.10)$$

erfüllen, wenn man aus der Schar der möglichen Lösungen, die (1.9) genügen, diejenige auswählt, die (1.10) erfüllt.

**Patentansprüche**

1. Verfahren zum Ermitteln der Position des distalen Endes (3) eines Knochenführungsdrahtes (1), bei dem

   - mit Hilfe eines medizintechnischen, optischen Tracking- und Navigationssystems (10) und einer Referenzvorrichtung (4) am proximalen Ende (2) des Führungsdrahtes die Position und Ausrichtung des proximalen Endes (2) des Führungsdrahtes (1) ermittelt wird;
   - mit Hilfe des medizintechnischen, optischen Tracking- und Navigationssystems (10) und einer Referenzvorrichtung (5) an einem Knochen (6), in dessen Röhrenkanal sich das distale Ende (3) des Führungsdrahtes befindet, die Ausrichtung des Röhrenkanals im Knochen (6) ermittelt wird; und bei dem

   (a) mittels der Randbedingungen für den Führungsdraht-Verlauf, die durch die Position und Ausrichtung des proximalen Endes (2) und die Ausrichtung des Röhrenkanals im Knochen (6) gegeben werden, und
   (b) unter Berücksichtigung der körperlichen Eigenschaften des Führungsdrahtes (1) computergestützt die Position des distalen Endes (3) des Knochenführungsdrahtes (1) ermittelt wird.

2. Verfahren nach Anspruch 1, bei dem die computergestützte Positionsermittlung durch den Computer des Tracking- und Navigationssystems (10) durchgeführt wird.

3. Bildunterstützungsverfahren für die Verwendung eines Knochenführungsdrahtes (1), bei dem mit Hilfe eines Verfahrens nach Anspruch 1 oder 2 die Position des distalen Endes (3) des Knochenführungsdrahtes (1) ermittelt wird, und bei dem diese Position auf einer Bildausgabe des Tracking- und Navigationssystems (10) in Relation zur Knochenanordnung ausgegeben wird.

4. Vorrichtung zum Ermitteln der Position des distalen Endes (3) eines Knochenführungsdrahtes (1), mit einem medizintechnischen, optischen Tracking- und Navigationssystems (10), mit einer Referenzvorrichtung (4) am proximalen Ende (2) des Führungsdrahtes und mit einer Referenzvorrichtung (5) an einem Knochen (6), in dessen Röhrenkanal sich das distale Ende (3) des Führungsdrahtes befindet, und mit einem Computer, der

   (a) mittels der Randbedingungen für den Führungsdraht-Verlauf, die durch die Position und Ausrichtung des proximalen Endes (2) und die Ausrichtung des Röhrenkanals im Knochen (6) gegeben werden, und
(b) unter Berücksichtigung der körperlichen Eigenschaften des Führungsdrahtes (1) die Position des distalen Endes (3) des Knochenführungsdrahtes (1) ermittelt.

**5.** Vorrichtung nach Anspruch 4, **dadurch gekennzeichnet, dass** der Computer der Computer des Tracking- und Navigationssystems (10) ist.

**6.** Vorrichtung nach Anspruch 4 oder 5, **dadurch gekennzeichnet, dass** das Tracking- und Navigationssystem (10) eine Bildausgabe aufweist, welche die ermittelte Position des distalen Endes in Relation zur Knochenanordnung ausgibt.

**7.** Programm, das, wenn es auf einem Computer läuft oder in einem Computer geladen ist, den Computer veranlasst, ein Verfahren gemäß einem der Ansprüche 1 bis 3 durchzuführen.

**8.** Computerprogramm-Speichermedium, das ein Programm nach Anspruch 7 aufweist.


**Claims**

**1.** A method for identifying the position of the distal end (3) of a bone guide wire (1), wherein:

- the position and orientation of the proximal end (2) of the guide wire (1) is identified with the aid of a medical, optical tracking and navigation system (10) and a reference device (4) on the proximal end (2) of the guide wire;
- the orientation of the tubular channel in a bone (6), in which the distal end (3) of the guide wire is located, is identified with the aid of the medical, optical tracking and navigation system (10) and a reference device (5) on the bone (6); and wherein

(a) by means of the ancillary conditions for the course of the guide wire, which are given by the position and orientation of the proximal end (2) and the orientation of the tubular channel in the bone (6), and
(b) by taking into account the physical properties of the guide wire (1),

the position of the distal end (3) of the bone guide wire (1) is identified with the assistance of a computer.

**2.** The method according to claim 1, wherein the positions are identified with the assistance of a computer using the computer of the tracking and navigation system (10).

**3.** An image support method for using a bone guide wire (1), wherein the position of the distal end (3) of the bone guide wire (1) is identified with the aid of a method according to claim 1 or 2, and wherein this position is outputted on an image output of the tracking and navigation system (10), in relation to the bone arrangement.

**4.** A device for identifying the position of the distal end (3) of a bone guide wire (1), comprising a medical, optical tracking and navigation system (10), a reference device (4) on the proximal end (2) of the guide wire and a reference device (5) on a bone (6) in the tubular channel of which the distal end (3) of the guide wire is located, and comprising a computer, which

(a) by means of the ancillary conditions for the course of the guide wire, which are given by the position and orientation of the proximal end (2) and the orientation of the tubular channel in the bone (6), and
(b) by taking into account the physical properties of the guide wire (1),

identifies the position of the distal end (3) of the bone guide wire (1).

**5.** The device according to claim 4, **characterised in that** the computer is the computer of the tracking and navigation system (10).

**6.** The device according to claim 4 or 5, **characterised in that** the tracking and navigation system (10) comprises an image output which outputs the identified position of the distal end in relation to the bone arrangement.

**7.** A program which, when running on a computer or loaded on a computer, causes the computer to carry out a method in accordance with any one of claims 1 to 3.

8. A computer program storage medium which comprises a program according to claim 7.

**Revendications**

1. Procédé pour déterminer la position de l'extrémité distale (3) d'un fil de guidage osseux (1), comportant les étapes consistant à :

   - déterminer, en utilisant un système de repérage et de navigation optique médical (10) et un dispositif de référence (4) sur l'extrémité proximale (2) du fil de guidage, la position et l'orientation de l'extrémité proximale (2) du fil de guidage (1),
   - déterminer, en utilisant le système de repérage et de navigation optique médical (10) et un dispositif de référence (5) sur un os (6) dans le canal tubulaire duquel se trouve l'extrémité distale (3) du fil de guidage, l'orientation du canal tubulaire dans l'os (6), et dans lequel

   la position de l'extrémité distale (3) du fil de guidage osseux (1) est déterminée à l'aide d'un ordinateur

   a) en utilisant des conditions limites pour le cheminement du fil de guidage qui sont données par la position et l'orientation de l'extrémité proximale (2) et par l'orientation du canal tubulaire dans l'os (6), et
   b) en tenant compte des caractéristiques corporelles du fil de guidage (1).

2. Procédé selon la revendication 1, dans lequel la détermination de position assistée par ordinateur est effectuée par l'ordinateur du système de repérage et de navigation (10).

3. Procédé de support d'image pour l'utilisation d'un fil de guidage osseux (1), dans lequel la position de l'extrémité distale (3) du fil de guidage osseux (1) est déterminée à l'aide d'un procédé selon la revendication 1 ou 2, et dans lequel cette position est générée à partir d'une sortie d'image du système de repérage et de navigation (10) par rapport à la disposition des os.

4. Dispositif pour déterminer la position de l'extrémité distale (3) d'un fil de guidage osseux (1), comportant un système de repérage et de navigation optique médical (10), un dispositif de référence (4) sur l'extrémité proximale (2) du fil de guidage et un dispositif de référence (5) sur un os (6) dans le canal tubulaire duquel se trouve l'extrémité distale (3) du fil de guidage, et un ordinateur qui détermine la position de l'extrémité distale (3) du fil de guidage osseux (1)

   (a) en utilisant des conditions limites pour le cheminement du fil de guidage qui sont données par la position et l'orientation de l'extrémité proximale (2) et par l'orientation du canal tubulaire dans l'os (6), et
   (b) en tenant compte des caractéristiques corporelles du fil de guidage (1).

5. Dispositif selon la revendication 4, **caractérisé en ce que** l'ordinateur est l'ordinateur du système de repérage et de navigation (10).

6. Dispositif selon la revendication 4 ou 5, **caractérisé en ce que** le système de repérage et de navigation (10) comporte une sortie d'image qui génère la position déterminée de l'extrémité distale par rapport à la disposition des os.

7. Programme qui, lorsqu'il s'exécute sur un ordinateur ou lorsqu'il est chargé dans un ordinateur, amène l'ordinateur à mettre en oeuvre un procédé selon l'une quelconque des revendications 1 à 3.

8. Support de mémorisation de programme informatique comportant un programme selon la revendication 7.

$\vec{r}_3, d\vec{r}_3/dl$

$\vec{r}_2, d\vec{r}_2/dl$

Fig. 1

$r = r_0$
$r' = r'_0$

$r' = r'_1$

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6104944 A
- US 6783536 B2
- DE 19639615 A1
- DE 10118570 A1